(19) Europäisches Patentamt
European Patent Office
Office européen des brevets

(11) Publication number: **0 098 032 A2**

(12) # EUROPEAN PATENT APPLICATION

(21) Application number: 83301984.7

(22) Date of filing: 08.04.83

(51) Int. Cl.³: C 07 D 501/20
C 07 D 499/68, A 61 K 31/545
A 61 K 31/43
//C07D261/18

(30) Priority: 12.04.82 JP 59611/82

(43) Date of publication of application: 11.01.84 Bulletin 84/2

(84) Designated Contracting States: DE FR GB

(71) Applicant: AJINOMOTO CO., INC.
5-8, Kyobashi 1-chome, Chuo-ku
Tokyo 104(JP)

(72) Inventor: Iwagami, Hisao
No. 2-20-8, Kannon Kawasaki-ku
Kawasaki-shi Kanagawa-ken(JP)

(72) Inventor: Nakanishi, Eiji
No. 2-20-8, Kannon Kawasaki-ku
Kawasaki-shi Kanagawa-ken(JP)

(72) Inventor: Sasaki, Yukio
No. 2-20-8, Kannon Kawasaki-ku
Kawasaki-shi Kanagawa-ken(JP)

(72) Inventor: Yamanaka, Shigeru
No. 3-40-13, Ooka Minami-ku
Yokohama-shi Kanagawa-ken(JP)

(72) Inventor: Yasuda, Naohiko
No. 2-36-2, Hairando
Yokosuka-shi Kanagawa-ken(JP)

(74) Representative: Bond, Bentley George et al,
HASELTINE LAKE & CO. Hazlitt House 28 Southampton Buildings Chancery Lane
London, WC2A 1AT(GB)

(54) **Beta-lactam derivatives and their pharmaceutical preparations.**

(57) There are disclosed 5-substituted-3-isoxazolecarboxylic acid derivatives having the general formula:

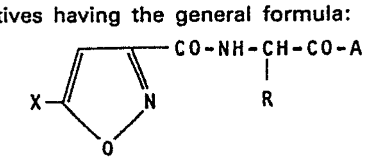

wherein X is a phenyl group, a thienyl group a furyl group or a pyridyl group, each of which groups optionally contains at least one substituent group, R is a phenyl group or a hydroxyphenyl group, and A is the following group:

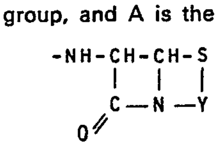

wherein Y is;

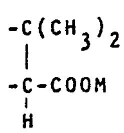
or
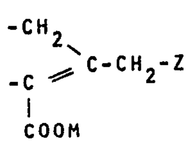

(the carbon atom of Y to which the carboxyl group is attached, being attached to the nitrogen atom of A) wherein M is a hydrogen atom or a substituent group, and Z is a hydrogen atom, a hydroxy group, an acyloxy group, a carbamoyloxy group, an aromatic heterocyclethio group or an aromatic nitrogen-containing heterocycle quarternary ammonium group. The derivatives are useful as antibiotics.

EP 0 098 032 A2

TITLE MODIFIED
see front page



5-SUBSTITUTED-3-ISOXAZOLECARBOXYLIC ACID DERIVATIVES

The present invention relates to novel 5-substituted-3-isoxazolecarboxylic acid derivatives, which can be used as antibiotics for treating infectious diseases of human beings and animals caused by Gram-positive bacteria, Gram-negative bacteria, especially glucose non-fermetative Gram-negative rods, and anaerobic bacteria.

We have succeeded in synthesising novel 5-substituted-3-isoxazolecarboxylic acid derivatives having the following general formula:

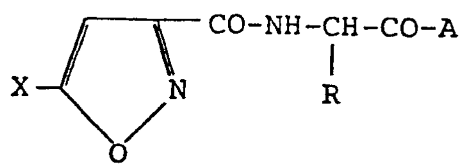

(I)

and have found that such novel compounds have a broad spectrum of antibacterial activity against Gram-positive and Gram-negative bacteria, and furthermore have a marked antibacterial activity against Pseudomonas aeruginosa and other glucose non-fermentative Gram-negative rods and anaerobic bacteria, and therefore can be used as antibiotics.

In the above formula (I), X in any either case is a phenyl group, a thienyl group, a furyl group or a pyridyl group, which may have at least one

substituent group. R is a phenyl group or a hydroxyphenyl group.

In the above formula (I), when X is a group having at least one substituent group, examples of the substituent groups are halogen atoms such as chlorine atoms, hydroxy groups, lower alkyl groups, lower alkyloxy groups, amino groups, acylamino groups and nitro groups.

The term "lower" herein usually means a group containing up to 4 carbon atoms, but includes a group containing up to 10 carbon atoms such as a group containing up to 8 carbon atoms or up to 6 carbon toms.

Amino acids constituting 5-substituted-isoxazole-3-carboxylic acid derivatives of the present invention, for example, α-phenyl-glycine (in the above formula I, R is aphenyl group), α-4-hydroxyphenylglycine (in the above formula I, R is a 4-hydroxyphenyl group), may be in the L-form, the D-form or the DL-form. Regarding antibacterial activity, the D-form is preferred in many cases.

In the above formula (I), A is a group having the following general formula:

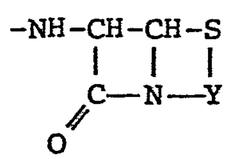

wherein Y is:

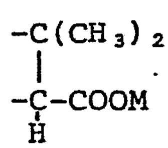

or

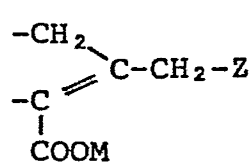

The carbon atom of Y to which the carboxyl group is attached, is attached to the nitrogen atom of A.

The radical M is a hydrogen atom or a non-toxic substituent group, and Z is a hydrogen atom, a hydroxy group, a acyloxy group, a carbamoyloxy group, an aromatic heterocycle-thio group, or aromatic nitrogen-containing heterocycle quarternary ammonium group.

An example of an acyloxy group is the acetoxy group. Examples of aromatic heterocycle-thio groups are the 5-(1-methyltetrazolyl)thio group and the 2-(1,3,4-thiadiazolyl)thio group. Examples of the aromatic nitrogen-containing heterocycle tertiary ammonium groups are pyridinium, quinolinium and picolinium groups, which groups may have at least one substituent group.

The 5-substituted-isoxazole-3-carboxylic acid derivatives of the present invention include derivatives of the above formula (I) wherein the hydrogen atom of the carboxyl group in Y is substituted by a metal such as sodium, potassium, calcium, aluminum, or by an ammonium radical such as triethylammonium, procaine, dibenzylammonium or N-benzyl-β-phenethylammonium. Thus, the derivative is in the salt form or is in the hydrate form. In this case a pharmaceutically non-toxic substituent group is employed.

It is known that penicillins having an amino group in the α-position and cephalosporins having the general formula:

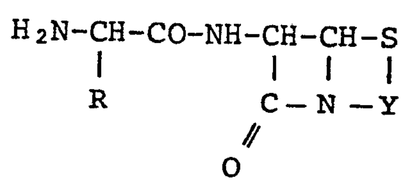

wherein R and Y have the same meanings as above, have an antibacterial activity against not only Gram-positive bacteria but also Gram-negative bacteria. However, they have the disadvantage that they do not have an effective antibacterial activity against <u>Pseudomonas</u>

aeruginosa and other glucose non-fermentative Gram-negative rods and anaerobic bacteria, causing clinically serious infectious diseases of human beings and animals.

The 5-substituted-isoxazole-3-carboxylic acid derivatives of the present invention have a broad spectrum of antibacterial activity against Gram-positive and Gram-negative bacteria, and furthermore have a marked antibacterial activity against Pseudomonas aeruginosa and other glucose non-fermentative Gram-negative rods and anaerobic bacteria, and therefore are useful compounds.

In a preferred method of producing the compounds of the present invention, α-aminopenicillins and cephalosporings having the general formula:

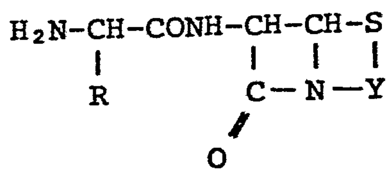

are reacted with 5-substituted-isoxazole-3-carboxylic acids having the general formula:

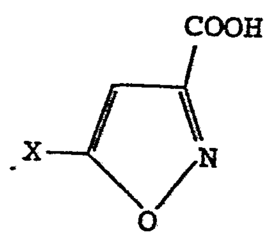

or reactive acid derivatives thereof for condensation. In another preferred method, penicillins and cephalosporins having the general formula:

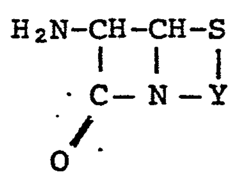

are reacted with 5-substituted-isoxazole-3-carboxylic acids having the general formula:

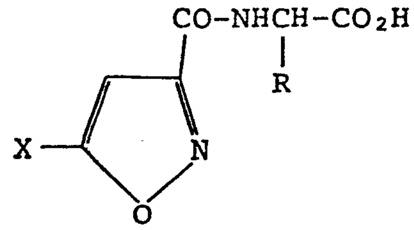

or reactive acid derivatives thereof for condensation.

For the above condensation reaction, a condensation reaction which is known as such can be employed. The radicals R, X and Y are as described above.

Examples of suitable reactive acid derivatives are acid halides, mixed anhydrides, activated amido derivatives and activated esters. It is preferred to use a derivative selected from acid chloride, acid azide, dialkylphosphoric acid mixed anhydride, phenylphosphoric acid mixed anhydride, diphenylphosphoric acid mixed anhydride, dibenzylphosphoric acid mixed anhydride, halogenized phosphoric acid mixed anhydride, dialkylphosphorous acid mixed anhydride, sulphurous acid mixed anhydride, thiosulphuric acid mixed anhydride, sulphuric acid mixed anhydride, alkylcarbonic acid mixed anhydride, fatty acid such as pivalic acid, pentanoic acid, isopentanoic acid, 2-ethylbutanoic acid, trichloroacetic acid, mixed anhydrides, aromatic carboxylic acids such as benzoic acid, p-methylbenzoic acid, mixed anhydride, acids anhydride, acid amides with imidazole, 4-substituted imidazole, dimethylpyrazole, triazole, tetrazole, esters such as cyanomethylester, methoxymethylester, vinylester, propargylester, p-nitrophenylester, 2,4-dinitrophenylester, trichlorophenylester, pentachlorophenylester, methane sulphonylphenylester, phenylazophenylester, phenylthioester, p-nitrophenylthioester, p-cresylthioester, carboxymethylthioester, pyranyl ester, pyridyl ester, piperidyl ester, 8-quinolilthio ester, ester with N,N-dimethylhydroxylamine, 1-hydroxy-2-(1H)-pyridone, N-hydroxy succinimide or N-hydroxy phthalimide.

The reactive acid derivatives are produced, for example, as follows. An acid chloride is synthesised by reacting the above mentioned 5-substituted-isoxazole-3-carboxylic acid or a derivative thereof with, for example, thionylchloride or phosphorus pentachloride. An activated ester such as 2,4-dinitrophenylester is obtained by reacting with 2,4-dinitrophenol in the presence of a condensing agent such as dicyclohexylcarbodiimide.

The reaction of the above mentioned reactive acid derivatives with the above mentioned penicillins or cephalosporins may be carried out in the presence of base such as alkali metal hydrogencarbonate, alkali metal carbonate, trialkylamine or pyridine. When a solvent is used, the solvent is preferably water, acetone, dioxane, acetonitrile, chloroform, methylene chloride, tetrahydrofuran or N,N-dimethyl formamide (DMF). A hydrophilic organic solvent can be used in admixture with water. The reaction is usually carried out with cooling or at room temperature.

In the reaction with the reactive acid derivative, it is also possible to react with an ester to which the carboxyl group in Y of the above general formula has been converted, for example, t-butylester, benzylester, silylester or trichloroethylester. These ester groups may be removed by a conventional method after the reaction to obtain the carboxyl group in free form.

The compounds of the above general formula wherein A has a cephalosporin skeleton and Z is aromatic heterocycle-thio group of a quarternary ammonium group, are obtained, for example, by synthesizing cephalosporins wherein Z is an acetoxy group, and thereafter substituting the acetoxy group by the aromatic heterocycle-thio group or the quarternary ammonium group.

Regarding the compounds of the above general

formula wherein the substituent group of X is a substituent group such as amino or acyl amino, a compound having an amino group as the substituent group is obtained, for example, by synthesizing penicillins or cephalosporins having a nitro group as the substituent group, and thereafter reducing it in the presence of a catalyst. Furthermore, by acylating the amino group of the resulting compound with an acylating agent such as an acyl halide, the required compound having an acylamino gorup as the substituent group is obtained.

The reaction product may be isolated by using conventional isolating methods such as extraction, column chromatography and recrystallization.

Thus 5-substituted-isoxazole-3-carboxylic acid derivatives may be converted to non-toxic salts such as alkali metal salts and ammonium salts, and to organic base salts by conventional salt-forming methods. These salts are preferable in, for example, the preparation of drugs since they can be dissolved in water.

The present invention will now be illustrated by the following Examples.

Example 1

A mixture of 5-(4-methylphenyl)-3-isoxazolecarboxylic acid (0.97 g, 5 m mole) and thionyl chloride (10 ml) was stirred at 80°C for 4 hours. After evaporating the mixture, dry benzene (10 ml) was added and the mixture was concentrated *in vacuo* again to give the corresponding acid chloride as the residue. This residue was dissolved in dry acetonitrile (15 ml).

Ampicillin trihydrate (1.81 g, 4.5 m mole) was suspended in a mixture of water (25 ml) and acetonitrile (10 ml), and the pH of the suspension was adjusted carefully to 8.5 with 2N NaOH under ice-cooling. To this solution, the acid chloride solution previously

prepared was added dropwise with stirring and ice-cooling. After the addition, the solution was stirred for 1 hour under ice-cooling and for 1 hour at room temperature. During the reaction, the pH of the mixture was kept at 7.5 to 8.0 with 2N NaOH and 2N HCl. After adding water (15 ml), the mixture was evaporated *in vacuo* at 30°C in order to remove acetonitrile. The water solution was covered with ethylacetate (100 ml) and the pH of water phase was brought to 1.5 with 2N HCl. The organic layer was separated and the water layer was extracted with ethyl acetate (100 ml). The combined organic layers were dried and evaporated to a syrup *in vacuo*. The residue was triturated with the mixture of ether-petroether (1:1). The triturated material was collected by filtration and dried under vacuum to give 1.6g of α-[5-(4-methylphenyl)-3-isoxazolecarboxamido]-benzylpenicillin.

IR spectrum (Nujol): 1780 $cm^{-1}$ (β-lactam);

NMR spectrum (DMSO-$d_6$): δ 1.44 (3H,S,-$CH_3$), 1.55 (3H,S,-$CH_3$), 2.36 (3H, S, $CH_3$-Ph), 4.20 (1H, S, $C_3$-H), 5.30-6.06 (3H, m, $C_5$-H, $C_6$-H and Ph-CH-CO), 6.75 (2H, d, 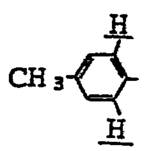 ), δ 7.17–7.60 (8H, m, Ph-H, 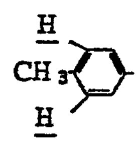 and isoxazole $C_4$-H).

Examples 2 to 10

The compounds given in Table 2 were synthesized by the method as described in Example 1.

Table 1

| Example No. | Object Compound | IR ($cm^{-1}$) $\nu_{C=O}$ (β-lactam) | NMR δ value; solvent |
|---|---|---|---|
| 2 | D-α-(5-phenylisoxazole-3-carboxamido)-benzylpenicillin sodium salt | 1770 | |
| 3 | D-α-(5-m-anisylisoxazole-3-carboxamido)-benzylpenicillin sodium salt | 1767 | 1.40(S,3H), 1.49(S,3H) 3.78(S,3H), 3.90(S,1H) 5.20∿5.50(m,2H) 5.87(d,1H), 6.80∿7.60 (m,10H); DMSO-$d_6$ |
| 4 | D-α-(5-m-nitrophenylisoxazole-3-carboxamido)-benzylpenicillin sodium salt | 1770 | |
| 5 | D-α-(5-p-methylphenylisoxazole-3-carboxamido)-p-hydroxybenzylpenicillin sodium salt | 1775 | 1.42(S,3H), 1.52(S,3H) 2.33(S,3H), 3.88(S,1H) 5.22∿5.53(m,2H) 5.72(d,1H), 6.68(d,2H); DMSO-$d_6$ |
| 7 | D-α-[5-(2-pyridyl)-isoxazole-3-carboxamido]-benzylpenicillin sodium salt | 1770 | |
| 8 | D-α-[5-(2-thienyl)-isoxazole-3-carboxamido]-benzylpenicillin sodium salt | 1773 | |
| 9 | D-α-(5-m-chlorophenylisoxazole-3-carboxyamido) benzylpenicillin | 1780 | |
| 10 | D-α-(5-m-aminophenylisoxazole-3-carboxamido)-benzylpenicillin | 1780 | |

Example 11

A mixture of 5-(m-anisyl)-isoxazole-3-carboxylic acid (2.70 g, 12.3 m mole) and thionyl chloride (25 ml) was stirred while heating at 80°C for 2 hours. After completion of the reaction, the solution was concentration <u>in vacuo</u> to remove thionyl chloride. To the thus obtained residue, dry benzene (25 ml) was added and the mixture was concentrated <u>in vacuo</u> again to give a solid material. The residue thus obtained, namely 5-(m-anisyl)-isoxazole-3-carboxylic acid chloride, was dissolved in acetonitrile (48 ml).

In a separate reaction 7β-[D(-)-(α-amino)-phenylacetoamide]-3-acetoxymethyl-3-cephem-4-carboxylic acid (5.5 g, 13.6 m mole) was suspended in a mixture of water (70 ml) and acetonitrile (34 ml) under ice-cooling, and 2N NaOH was added dropwise thereto to convert the mixture into a homogeneous solution. The pH of the solution was 8.5. To this solution, the acid chloride acetonitrile solution previously prepared was added dropwise with stirring and ice-cooling. During the reaction, the pH of the mixture was kept at 7.5 to 8.0 with 6% HCl and sodium hydrogencarbonate saturated aqueous solution.

After the reaction, water (35 ml) was added to the reaction solution, and then acetonitrile was distilled off <u>in vacuo</u> and at not more than 30° C. The remaining aqueous solution was washed with ethyl acetate (180 ml). To the residual aqueous solution, ethyl acetate (230 ml) was added, and then 6% HCl was added dropwise while stirring to reduce the pH of the water layer to 2.0. The ethyl acetate layer was separated. The aqueous layer was extracted again with ethyl acetate (100 ml). The two ethyl acetate layers were combined together and dried over anhydrous magnesium sulphate.

The ethyl acetate solution was concentrated

at not more than 30°C, and, to the thus obtained residue, ethyl ether was added to form a powdered material. Thus obtained solid material was placed on filter paper and dried to obtain the desired product, namely 7β-[D-(-)-α-(5-(m-anisyl)-isoxazole-3-carboxamido)-α-phenylacetamido] cephalosporanic acid (4.59 g, 7.57 m mole; yield: 61.5%).

The product was added to a mixture of methanol (60 ml) and ethyl acetate (60 ml), and then, to the mixture while stirring at room temperature, sodium 2-ethyl hexanoate n-butanol solution (2M/l, 4.46 ml) was added, and stirring was carried out for 15 minutes. This solution was allowed to stand with ice-cooling for 4 hours to precipitate a solid material. The resulting solid material was placed on filter paper and dried to obtain the desired product, namely 7β-[D(-)-α-(5-(m-anizyl)isoxazole-3-carboxamido)-α-phenylacetoamido] cephalosporanic acid sodium salt (4.44 g, 7.07 m mole; yield: 93.4%).

IR spectrum (Nujol) :

$\nu_{C=o}$ (β-lactam) = 1760 $cm^{-1}$

$\nu_{C=o}$ ($\mathrm{O\overset{\overset{O}{\|}}{C}CH_3}$) = 1725 $cm^{-1}$

NMR spectrum (solvent: DMSO)

| | | |
|---|---|---|
| δ | 1.97 (S, 3H) | ($\underline{H_3}\mathrm{\overset{\overset{O}{\|}}{C}O}^-$) |
| | 3.08 — 3.48 (dd, 2H) | ($-CH_2-$(2-position)) |
| | 3.82 (S, 3H) | ($\underline{H_3}CO$— 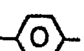 ) |
| | 4.62 — 5.15 (dd, 2H) | ($-\underline{CH_2}-\mathrm{O\overset{\overset{O}{\|}}{C}CH_3}$) |
| | 5.35 — 5.98 (m, 3H) | ($C\underline{H}$— 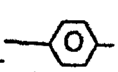 , -H (6-position), -H (7-position)) |
| | 6.95 — 7.60 (m, 10H) | (arom) |

Example 12

7β-[D(-)-α-(5-(m-anisyl)-isoxazolyl-3-carboxamido]cephalosporanic acid sodium salt as prepared in Example 11 (1.26 g, 2.0 m mole) was dissolved in phosphate buffer (20 ml, pH 6.4) and 1-methyl-5-mercapto-1H-tetrazole (258 mg, 2.22 m mole) was added thereto. In this case, the pH of the solution was reduced, and was adjusted to 6.4 with 2N NaOH. This solution was reacted at 60°C for 24 hours while stirring. After 5 hours of reaction, the pH was adjusted to 6.5 with 2N NaoH. After completion of the reaction, to the reaction solution, water (40 ml) was added and an insoluble material was separated by filtration. To the filtrate, with ice-cooling, 2N NaOH was added to adjust the pH to 7.0, and the mixture was washed with ethyl acetate (60 ml). To the aqueous layer, ethyl acetate (100 ml) was added, and 6% HCl was added thereto while stirring to adjust the pH to 2. An insoluble material was removed by filtration, and a solution having two layers was obtained. The aqueous layer was extracted again with ethyl acetate (100 ml). The ethyl acetate layers were combined together, washed with water, dried over anhydrous magnesium sulphate, and concentrated. To the residue, ethyl ether was added to convert it to a powder. The powered material was placed on filter paper and dried to obtain the desired product, namely 7β-[D(-)-α-(5-(m-anisyl)-isoxazole-3-carboxamido)-α-phenylacetamido]-3-(1-methyl-1H-tetrazole-5-yl) thiomethyl-3-cephem-4-carboxylic acid (315 mg, yield: 25.08%). This product was converted to a sodium salt in the same manner as in Example 11.

IR spectrum (Nujol) :

$\nu_{c=o}$(β-lactam)=1780 $cm^{-1}$

Example 13

7β-[D(-)-α-(5-(2-thienyl)-isoxazole-3-carbox-

amido)-α-phenyl-acetamido]-3-acetoxymethyl-3-cephem-4-carboxylic acid sodium salt was produced in the same manner as in Example 11.

IR spectrum (Nujol) :

$\nu_{c=o}$ (β-lactam) = 1775 $cm^{-1}$

Example 14

Isonicotinamido (244 mg, 2mM) and potassium iodide (8.3 g) were dissolved in water (11 ml), and then 7β-[D(-)-α-(5-(2-thienyl)-isoxazole-3-carboxamido)-α-phenyl-acetamido]-3-acetoxy-methyl-3-cephem-4-carboxylic acid sodium salt as synthesized in Example 13 (582 mg) was added thereto. The mixture was stirred while heating at 70°C for 2 hours. After reaction, the reaction solution was cooled and treated with "XAD-2" produced by Rhöm & Haas Co. (500 ml). The desired product was eluted with water. The fractions containing the desired product were collected and freeze-dried to obtain the desired product, namely 7β-[D(-)-α-(5-(2-thienyl)-isoxazole-3-carboxamido)-α-phenyl-acetamido]-3(4-carbamoylpyridinium)methyl-3-cephem-4-carboxylate (4 mg).

IR spectrum (Nujol):

$\nu_{c=o}$ (β-lactam) = 1767 $cm^{-1}$

Antibacterial Activity

The antibacterial activities of some of the compounds produced by the above Examples against some microorganisms were determined. Some of the results are given in Table 2.

As is evident from Table 2, the spectrum of the compounds of the present invention for antibacterial activities against microorganisms is very wide, and the antibacterial activities are superior to the conventional standard antibiotics. Accordingly, the present invention is a remarkable improvement.

Table 2 Antibacterial Activity (MIC μg/ml)

| Example No. | 1 | 3 | 8 | Carbenicillin (Control) |
|---|---|---|---|---|
| *Staphylococcus aureus* | 0.10 ≦ | 0.20 | 0.10 ≦ | 0.78 |
| *Escherichia coli* | 12.5 | 12.5 | 12.5 | 12.5 |
| *Klebsiella pneumoniae* | 6.25 | 12.5 | 12.5 | > 100 |
| *Proteus mirabilis* | 0.78 | 0.39 | 0.78 | 0.78 |
| *Serratia marcescens* | 12.5 | 12.5 | 25 | > 100 |
| *Pseudomonas aeruginosa* | 3.13 | 6.25 | 6.25 | 25 |
| *Pseudomonas cepacia* | 3.13 | 3.13 | 6.25 | > 100 |
| *Pseudomonas maltophilia* | 6.25 | 12.5 | 12.5 | > 100 |
| *Alcaligenes faecalis* | 6.25 | 6.25 | 12.5 | > 100 |
| *Achromobacter xylosoxidans* | 1.56 | 3.13 | 3.13 | > 100 |
| *Bacteroides fragilis* | 6.25 | 12.5 | 12.5 | > 100 |

CLAIMS

1. A 5-substituted-3-isoxazolecarboxylic acid derivative having the general formula:

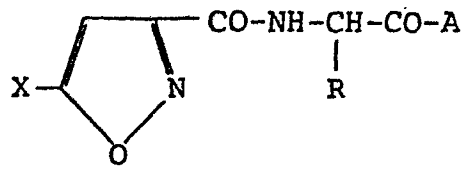

wherein X is a phenyl group, a thienyl group, a furyl group of a pyridyl group, each of which groups optionally contains at least one substituent group, R is a phenyl group or a hydroxyphenyl group, and A is the following group:

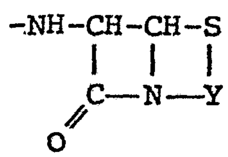

wherein Y is:

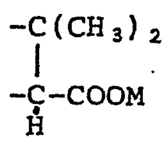

or

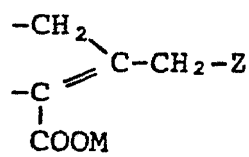

(the carbon atom of Y to which the carboxyl group is attached, being attached to the nitrogen atom of A) wherein M is a hydrogen atom or a substituent group, and Z is a hydrogen atom, a hydroxy group, an acyloxy group, a carbamoyloxy group, an aromatic heterocyclethio group or an aromatic nitrogen-containing heterocycle quarternary ammonium group.

2. A derivative as claimed in claim 1, wherein the substituent group which X contains is a halogen atom, a hydroxy group, a lower alkyl group, a lower alkyloxy group, an amino group, an acylamino group or a nitro group.

3. A derivative as claimed in claim 1 or 2, wherein the amino acid constituting the derivative is in the D-form.

4. A pharmaceutical preparation comprising a derivative as claimed in claim 1, 2 or 3, and a pharmaceutically-acceptable carrier or diluent.